# EUROPÄISCHE PATENTSCHRIFT

(11) **EP 1 904 855 B1**
(45) Veröffentlichungstag und Bekanntmachung des Hinweises auf die Patenterteilung: **06.10.2010**
(21) Anmeldenummer: 06776308.6
(22) Anmeldetag: 19.07.2006
(51) Int. Cl.: G01N 33/68, G01N 33/543, G01N 33/74

(54) **LIQUORDIAGNOSTISCHES IN VITRO VERFAHREN ZUR DIAGNOSE VON DEMENZ-ERKRANKUNGEN UND NEUROINFLAMMATORISCHEN ERKRANKUNGEN**
CSF DIAGNOSTIC IN VITRO METHOD FOR DIAGNOSIS OF DEMENTIA AND NEUROINFLAMMATORY DISEASES
PROCEDE DE DIAGNOSTIC DU LIQUIDE CEREBRO-SPINAL IN VITRO DESTINE AU DIAGNOSTIC DE DEMENCE ET DE MALADIES NEURO-INFLAMMATOIRES

(30) Priorität: 21.07.2005 DE 102005034174
(43) Veröffentlichungstag der Anmeldung: 02.04.2008
(73) Patentinhaber: B.R.A.H.M.S GmbH, 16761 Hennigsdorf (DE)
(72) Erfinder: BERGMANN, Andreas, 12351 Berlin (DE); ERNST, Andrea, 16761 Hennigsdorf (DE); HAMPEL, Harald, 80331 München (DE)
(74) Vertreter: Andrae, Steffen
(86) Internationale Anmeldenummer: PCT/EP2006/007141
(87) Internationale Veröffentlichungsnummer: WO 2007/009789

(56) Entgegenhaltungen:
- EP-A- 1 110 970
- JEREB M ET AL: "Predictive value of serum and cerebrospinal fluid procalcitonin levels for the diagnosis of bacterial meningitis" INFECTION, Bd. 29, Nr. 4, August 2001 (2001-08), Seiten 209-212, XP002400924 ISSN: 0300-8126 in der Anmeldung erwähnt
- MORGENTHALER N G ET AL: "Sensitive immunoluminometric assay for the detection of procalcitonin" CLINICAL CHEMISTRY 2002 UNITED STATES, Bd. 48, Nr. 5, 2002, Seiten 788-790, XP002400925 ISSN: 0009-9147 in der Anmeldung erwähnt
- MORGENTHALER NILS G ET AL: "Detection of procalcitonin (PCT) in healthy controls and patients with local infection by a sensitive ILMA." CLINICAL LABORATORY. 2002, Bd. 48, Nr. 5-6, 2002, Seiten 263-270, XP009072951 ISSN: 1433-6510
- BERGMANN ET AL: "P2-149" ALZHEIMER'S & DEMENTIA: THE JOURNAL OF THE ALZHEIMER'S ASSOCIATION, ELSEVIER, NEW YORK, NY, US, Bd. 2, Nr. 3, 5. Juli 2006 (2006-07-05), Seite S277, XP005578548 ISSN: 1552-5260
- STEINBACH GERALD ET AL: "Multicenter evaluation of a new immunoassay for procalcitonin measurement on the Kryptor(R) System" CLINICAL CHEMISTRY AND LABORATORY MEDICINE, Bd. 42, Nr. 4, 2004, Seiten 440-449, XP009072935 ISSN: 1434-6621
- VAN ROSSUM A ET AL: "Procalcitonin as an early marker of infection in neonates and children" LANCET INFECTIOUS DISEASES, US, Bd. 4, Nr. 10, Oktober 2004 (2004-10), Seiten 620-630, XP004808552 ISSN: 1473-3099

## Beschreibung

Die vorliegende Erfindung betrifft ein neues liquordiagnostisches in vitro Verfahren zur Diagnose von Demenz-Erkrankungen und chronischen neuroinflammatorischen Erkrankungen nicht-infektiöser Ätiologie gemäß Anspruch 1.

Im Rahmen der vorliegenden Erfindung wird dabei der Begriff "Diagnose" als Oberbegriff für medizinische Bestimmungen gebraucht, denen je nach dem klinischen Zustand des Patienten, bei dem die Bestimmung durchgeführt wird, unterschiedliche Fragestellungen zugrunde liegen können und die insbesondere der Erkennung und Früherkennung, der Bestimmung des Schweregrads und der Verlaufsbeurteilung, auch der therapiebegleitenden Verlaufsbeurteilung, und der Prognose des zukünftigen Verlaufs einer Erkrankung dienen.

Das erfindungsgemäße Verfahren ist ein liquordiagnostisches *in vitro* Verfahren. Unter einem liquordiagnostischen Verfahren wird ein Verfahren verstanden, das üblicherweise im Rahmen der Diagnostik von neurologischen Erkrankungen.durchgeführt wird und bei dem die Bestimmung einer für Diagnosezwecke aussagekräftigen Eigenschaft des sogenannten Liquor cerebrospinalis (Abkürzung CSF, abgeleitet vom englischen Fachbegriff "cerebrospinal fluid") erfolgt. Im Falle der vorliegenden Erfindung ist die bestimmte Eigenschaft der immundiagnostisch bestimmbare Gehalt eines Biomoleküls im CSF.

Die Erkrankungen, die gemäß der vorliegenden Erfindung diagnostiziert werden, sind insbesondere präsenile Demenz-Erkrankungen, wie sie in der vorliegenden Anmeldung noch näher diskutiert werden, und weitere chronische neuroinflammatorische Erkrankungen nichtinfektiöser Ätiologie.

Als Demenz-Erkrankungen (Dementia) werden generell Krankheiten bezeichnet, für die ein gemeinsames Merkmal der Verlust erworbener intellektueller Fähigkeiten, v.a. des Gedächtnisses, und des normalen Persönlichkeitsniveaus als Folge von Hirnschädigungen ist. Demenzerkrankungen sind in der Regel sich relativ langsam entwickelnde Krankheiten von chronischem Charakter. Treten Demenzerscheinungen vor dem hohen Alter im mittleren Lebensalter auf, werden sie als präsenile Demenz-Erkrankungen bezeichnet, und bei diesen unterscheidet man auf der Basis der für sie typischen Symptome und hirnpathologischen Veränderungen insbesondere die folgenden vier Erkrankungen bzw. Gruppen von Erkrankungen:

Die Alzheimer Demenz (AD) (Alzheimer Krankheit; Morbus Alzheimer) ist die häufigste neurodegenerative Demenz-Erkrankung und macht 2/3 aller Demenzfälle aus. AD zeichnet sich durch drei wichtige pathologische Merkmale aus: die Bildung von Amyloid-Plaques und neurofibrillären Bündeln sowie den Verlust an Nervenzellen (Übersicht s. 24; Literaturangaben in der Beschreibung in Form von Zahlen beziehen sich auf die der Beschreibung folgende Literaturliste). Amyloid-Plaques bestehen aus extraneuronalen Aggregaten des Amyloid-ß-Proteins, während die Neurofibrillenbündel hauptsächlich Tau-Protein und Neurofilamente enthalten. Es wird vermutet, dass die Plaque- und Neurofibrillenbildung die Ursache für das Absterben von Nervenzellen ist.

Die wichtigsten Symptome von AD sind zunehmende Merkfähigkeits- und Denkstörungen bei relativ lang anhaltender Gemütsansprechbarkeit, wobei diese Symptome von weiteren weniger spezifischen Störungen begleitet werden, die die Abgrenzung der AD von anderen Demenzformen erschweren.

Die Demenz mit Lewy-Körperchen (dementia with lewy-bodies: DLB) ist nach der Alzheimer Demenz die zweithäufigste Ursache für eine demenzielle Erkrankung (11; 18). Neuropathologisch ist die DLB durch das Auftreten von sogenannten LewyKörperchen im Hirnstamm und im Cortex charakterisiert. Diese Lewy-Körperchen bestehen überwiegend aus Aggregaten des präsynaptischen Proteins (α-Synuclein) und Ubiquitin. Die Lewy-Body-Pathologie kann in unterschiedlichem Ausmaß mit Alzheimer und Parkinson-typischen neuropathologischen Veränderungen assoziiert sein. So kommt es auch bei der DLB zur Bildung von beta-Amyloid und senilen Plaques, jedoch nicht zu Neurofibrillenbündeln (Übersicht s. 6). Lewy-Körperchen sind auch im Gehirn von Patienten mit Morbus Parkinson vorhanden, wenn auch in einer unterschiedlichen Verteilung (Übersicht s. 19).

Kernsymptome von DLB sind eine progrediente kognitive Störung, Verwirrtheitsepisoden mit fluktuierender Aufmerksamkeits- und Bewusstseinslage, Parkinsonismus, häufige Stürze und Synkopen (anfallsartige, kurz dauernde Bewusstlosigkeit) (17). Die Sensitivität und Spezifität der diagnostischen Kriterien (17) zeigen durchgehend eine hohe Spezifität, aber zum Teil eine sehr niedrige Sensitivität. Das bedeutet, dass die DLB im klinischen Alltag häufig nicht diagnostiziert wird. Insbesondere die Abgrenzung vom Morbus Alzheimer muss weiter verbessert werden.

Die Frontotemporale Demenz (FTD) wird auch als Pick'sche Krankheit bezeichnet und macht ca. 20% der präsenilen Demenzerkrankungen aus. FTD ist teilweise genetisch bedingt und zählt zu den sogenannten Tauopathien, die sich durch eine Über- oder Unterexpression eines Tauprotein-Subtyps (34) bzw. durch die Expression eines mutierten Tauproteins auszeichnen (23). Neuropathologisch kommt es zu einer lokalen Atrophie des frontalen und/oder temporalen Cortex sowie der Substantia Nigra und der Basalganglien. Dies hat unterschiedlich ausgeprägte Sprachstörungen, eine Wesensänderung sowie Verhaltensauffälligkeiten zur Folge. Insgesamt ist die FTD mit einer Sensitivität von 93% bei einer Spezifität von nur 23% unterdiagnostiziert, wobei die AD die häufigste Fehldiagnose darstellt (30).

Unter dem Begriff vaskuläre Demenz (vascular dementia; VAD) werden Erkrankungen zusammengefasst, bei denen eine Demenz aufgrund von Durchblutungsstörungen im Gehirn ausgelöst wird. Es gibt unterschiedliche Typen der VAD, von denen die Multi-Infarkt-Demenz (MID) und die subcorticale VAD (auch als Binswanger'sche Erkrankung bezeichnet) die häufigsten Formen darstellen.

Bei der Binswanger'schen Erkrankung handelt es sich um eine langsam progrediente demenzielle Entwicklung, die pathologisch durch cerebrovasculäre Läsionen in der weißen Hirnsubstanz charakterisiert ist. Klinisch resultiert dies in Verhaltensauffälligkeiten wie Agitation, Reizbarkeit, Depression und Euphorie sowie einer leichten Gedächtnisstörung (4).

Die Multi-Infarkt-Demenz entsteht allmählich als Folge von mehreren kleinen Schlaganfällen, auch als transiente ischämische Attacken (TIA) bezeichnet, die zum Untergang von Hirngewebe im Cortex und/oder subcortikalen Arealen führten (9). Die Schlaganfälle können auch gänzlich unbemerkt geblieben sein, die Demenz ist in diesem Falle die erste spürbare Folge. Bei Vorliegen einer MID kommt es zur stufenförmigen Abnahme kognitiver Fähigkeiten, verbunden mit schweren Depressionen, Stimmungsschwankungen und Epilepsie.

Eine Diagnose auf Demenz-Erkrankungen erfolgt heutzutage überwiegend auf der Basis neuropsychologischer Untersuchungen und der Beobachtung der Krankheitsentwicklung und ihres Verlaufs, unter Heranziehung von Ausschlusskriterien für bestimmte Demenzformen. Diese Untersuchungen liefern in sehr vielen Fällen mehrdeutige Ergebnisse, die die o.g. Zahlen für die unterdiagnostizierten Demenzformen oder unrichtig diagnostizierten Fälle erklären. Die krankheitstypischen Gehirnveränderungen können an lebenden Patienten naturgemäß nicht direkt festgestellt werden, und apparatemedizinische Untersuchungen der Gehirnfunktionen mittels z.B. Röntgen- oder Kernspin-Tomographie sind aufwändig und teuer.

Es besteht ein Bedarf nach ergänzenden Untersuchungsmethoden, die eine Demenzdiagnose ermöglichen und die insbesondere auch die Unterscheidung von verschiedenen Demenzformen mit ähnlichen oder verwaschenen klinischen Symptomen erleichtern, wobei eine immundiagnostische Bestimmung von Biomarkern einer geeigneten Spezifität und Sensitivität besonders wünschenswert wäre.

Die vorliegende Erfindung stellt eine solche Untersuchungsmethode in Form eines liquordiagnostischen *in vitro* Verfahrens zur Erkennung, zur Bestimmung des Schweregrads und zur Verlaufsbeurteilung und Prognose von Demenz-Erkrarlkungen und von chronischen neuroinflammatorischen Erkrankungen nicht-infektiöser Ätiologie und zur Durchführung im Rahmen der Differerltialdiagnostik von Demenz-Erkrankungen, wobei die Demenz-Erkrankungen ausgewählt sind aus einer Gruppe, die die Alzheimer Demenz (AD), Demenz mit Lewy-Körperchen (DLB), frontotemporale Demenz (FTD) und verschiedene Formen der vaskulären Demenz (VD) umfaßt, gemäß Anspruch 1 bereit, bei dem man in einer Probe des Liquor cerebrospinalis (CSF) eines Patienten, der an einer Demenz-Erkrankung oder chronischen neuroinflammatorischen Erkrankung nicht-infektiöser Ätiologie leidet oder bei dem Verdacht auf eine solche Erkrankung besteht, eine Bestimmung der Procalcitonin-Immunreaktivität (PCT-Immunreaktivität) durchführt und aus einer gemessenen PCT-Immunreaktivität, die über einem für gesunde Kontrollpersonen typischen Schwellenwert liegt, Schlüsse hinsichtlich des Vorliegens, des Typs, des Verlaufs, des Schweregrads oder des Erfolgs einer Therapie der Demenz-Erkrankung oder chronischen neuroinflammatorischen Erkrankung nicht-infektiöser Ätiologie zieht.

Insbesondere erfolgt die PCT-Bestimmung im CSF, wie in Anspruch 2 hervorgehoben, mit Hilfe eines hochsensitiven PCT-Immunoassays mit einer funktionalen Assay-Sensitivität (FAS) von besser als 100 ng PCT pro l (100 ng/l oder 100 pg/ml), insbesondere besser als 50 ng/l und besonders bevorzugt besser als 10 ng/l.

Vorteilhafte Ausgestaltungen eines Verfahrens gemäß den Ansprüchen 1 und 2 sind in den Unteransprüchen 3 bis 10 wiedergegeben.

Da die nachfolgend genauer beschriebenen Messungen gezeigt haben, dass man die PCT-Immunreaktivität in CSF mit einem hochsensitiven Immunoassay mit einer funktionalen Assay-Sensitivität (FAS), die erheblich besser ist als die der für die Sepsisdiagnostik verfügbaren kommerziellen PCT-Immunoassays, mit hoher Präzision und Zuverlässigkeit messen kann, während - wie nachfolgend erläutert wird - die vereinzelten bisherigen Versuche einer PCT-Bestimmung in CSF mit den bekannten Assays zu widersprüchlichen, wenig aussagekräftigen Ergebnissen führten, betrifft die Anmeldung außerdem ganz allgemein die Verwendung eines hochsensitiven Immunoassays zur Procalcitoninbestimmung mit einer funktionalen Assay-Sensitivität (FAS) von 50 ng/l und besser, insbesondere von 10 ng/l und besser, zur Bestimmung der Procalcitonin-Immunreaktivität im Liquor cerebrospinalis (CSF).

Als funktionale Assay-Sensitivität (FAS; auch funktionale Interassay-Sensitivität) wird eine Größe bezeichnet, die die Analytenkonzentration angibt, die mit der jeweiligen Methode mit einer Interassay-Präzision (einem Interassay-Variations-koeffizienten) von ≤ 20% gemessen wird (35).

Der vorliegenden Erfindung liegen Überlegungen der Erfinder zugrunde, zur Verbesserung der Differentialdiagnostik zur Unterscheidung verschiedener Formen der präsenilen Demenz bei der Erkenntnis anzusetzen, dass die bekannten, eingangs näher erläuterten Formen der präsenilen Demenz auch - in unterschiedlichem Ausmaß - von entzündlichen (inflammatorischen) Prozessen begleitet sind, die als wesentlich für die Entwicklung, die Symptome und den Verlauf der Demenzerkrankungen angesehen werden.

So ist Morbus Alzheimer u.a. durch das Auftreten chronischer lokaler Entzündungsreaktionen im Gehirn unter Beteiligung von verschiedenen inflammatorischen Proteinen wie Komplement-Faktoren, Akutphasen-Proteinen und proinflammatorischen Cytokinen gekennzeichnet (1; 30).

Inflammatorische Prozesse spielen auch eine Rolle bei der Entstehung von vaskulären Demenzen (VAD). Die Level von TNFα, TGFβ, IL-6 und GM-CSF (Granulozyten-Makrophagen-stimulierender Faktor) sind bei Patienten mit VAD deutlich erhöht (28; 29).

Es wird vermutet, dass sowohl bei der AD als auch bei der VAD eine ähnliche Cytokinproduktions-Kaskade als Antwort auf neuronale Schädigung in Gang gesetzt wird, obwohl die auslösenden Faktoren dieser beiden Neurodegenerationsformen unterschiedlich sind und zu unterschiedlichen neuropathologischen Veränderungen im Gehirn führen (28).

Auch bei DLB scheinen inflammatorische Prozesse eine Rolle zu spielen. So ist die Zahl der aktivierten Mikroglia-Zellen im Gehirn von Patienten mit DLB erhöht (15), und proinflammatorische Cytokine wie TNFα werden in bestimmten Hirnregionen wie der Amygdala und dem Hippocampus überexprimiert (13).

Für das Auftreten inflammatorischer Reaktionen im Gehirn von FTD-Patienten gibt es nur vereinzelte Hinweise. In einer Studie von Sjogren und Kollegen konnten in der cerebrospinalen Flüssigkeit von einigen FTD-Patienten signifikant erhöhte Konzentrationen des proinflammatorischen Cytokins TNFα und des anti-inflammatorischen Cytokins TGFβ gemessen werden (26).

Vor dem Hintergrund eines umfangreichen klinischen Materials und umfangreicher Erfahrungen im Hause der Anmelderin, die das Auftreten des Peptids Procalcitonin (PCT) im Serum und Plasma von Sepsispatienten und anderen Patienten betreffen, sahen es die Erfinder als lohnende Fragestellung an, ob sich Veränderungen von PCT-Konzentrationen, die sich in diagnostisch relevanter Weise zu Demenz-Erkrankungen und anderen neuroinflammatorischen Erkrankungen in Beziehung setzen lassen, im CSF feststellen lassen.

Procalcitonin (PCT) ist ein aus 116 Aminosäuren bestehendes Peptid, das zuerst als Vorläufer des wichtigen Hormons Calcitonin (Thyreocalcitonin) diskutiert wurde und dessen vollständige Aminosäuresequenz seit langem genauso bekannt ist wie die Details seines proteolytischen Abbaus, der zur Freisetzung des reifen Hormons Calcitonin und anderer kürzerer Peptide, darunter insbesondere das sogenannten Katacalcin (Procalcitonin 96-116) und ein N-terminales Peptids (N-Procalcitonin 1-57), führt, die hierin kurz als "PCT-Teilpeptide" bezeichnet werden. Wie z.B. in den Patenten EP 0 656 121 B1 bzw. US 5,639,617 und in (2) genauer erläutert wird, wird bei schweren bakteriellen Inflammationen mit systemischer Reaktion die Freisetzung von PCT in den Kreislauf induziert, wo es in sehr hohen, gut messbaren Mengen gefunden wird (2; s. auch die Übersichten in 22; 33; 3). Auf das in den genannten Patenten und Literaturstellen niedergelegte allgemeine Fachwissen wird zur Ergänzung der vorliegenden Beschreibung ausdrücklich verwiesen. Virale, Autoimmun- und allergische Erkrankungen führen dagegen nicht zu einer nennenswerten Erhöhung der PCT-Konzentration im Blut. PCT reflektiert den Schweregrad einer bakteriellen Infektion und wird als Marker für die Diagnose und das therapeutische Monitoring von Sepsis, schwerer Sepsis und septischem Schock bakteriellen Ursprungs verwendet (5; 7; 27; 32; 21).

Die Bestimmung von PCT läßt sich auch zu differentialdiagnostischen Zwecken nutzen, da sich anhand der messbaren PCT-Konzentrationen in Serum und Plasma entzündliche Erkrankungen infektiöser Ätiologie von solchen nicht-infektiöser Ätiologie unterscheiden lassen (vgl. auch EP 0 880 702 B1).

Die Bestimmung von PCT erfolgt, wie in den oben genannten Patenten und Literaturstellen beschrieben, geeigneter Weise mit Immunoassays vom Sandwichtyp unter Verwendung von zwei Antikörpern, die so an die Aminosäuresequenz des vollständigen PCT-Peptids binden, dass das vollständig unter Freisetzung von Calcitonin prozessierte PCT nicht erfasst wird, dagegen das gesamte unprozessierte PCT sowie gegebenenfalls auch solche längeren PCT-Teilpeptide, die beide Bindungsstellen für die im Assay verwendeten Antikörper aufweisen. Da die verwendeten Sandwichassays als solche nicht ausschließlich das vollständige unprozessierte PCT erkennen, wird im Rahmen der vorliegenden Anmeldung vorgezogen, statt von einer PCT-Bestimmung von der Bestimmung einer PCT-Immunreaktivität zu sprechen, wodurch der Anschein einer Festlegung auf eine ausschließliche Messung eines Moleküls mit der vollständigen PCT-Sequenz vermieden werden soll. Verallgemeinert läßt sich die Messung der PCT-Immunreaktivität als Messung mit einem Sandwich-Immunoassay unter Verwendung von zwei Antikörpern bezeichnen, die an solche Abschnitte des vollständigen PCT-Peptids binden, die bei der proteolytischen Prozessierung von PCT unter Bildung von Calcitonin auf verschiedenen der gebildeten PCT-Teilpeptide liegen oder die auf PCT-Teilpeptiden liegen, die die Calcitonin-Sequenz nicht umfassen.

Dass bei Sepsis in Serum oder Plasma nicht das vollständige PCT 1-116 bestimmt wird, sondern ein um zwei Aminosäuren verkürztes PCT 3-116, wird erläutert in EP 1 121 600 A1 bzw. EP 1 408 334 A1 oder US 6,756,483, auf die zur Ergänzung der vorliegenden Beschreibung verwiesen wird.

Zur Bestimmung der Procalcitonin-Immunreaktivitäten in Serum/Plasma existiert z.B. der kommerzielle Chemilumineszenz-Assay LUMItest^{®} PCT (B.R.A.H.M.S AG), der eine funktionalen Assay-Sensitivität (FAS) von 300 ng/l aufweist und auf die PCT-Bestimmung bei Sepsis zugeschnitten ist, wo sehr hohe PCT-Konzentrationen auftreten können. Für die PCT-Bestimmung mit einer höheren Sensitivität wurde in jüngerer Zeit ein abgewandelter Sandwichimmunoassay entwickelt, der mit einem affinitätsgereinigten polyklonalen Antikörper arbeitet und der in näheren Einzelheiten in (20) beschrieben wird und als LUMItest^{®} PCTsensitiv (B.R.A.H.M.S AG) erhältlich ist. Dieser Assay weist eine deutlich bessere FAS von 7 ng/l auf (20). Mit Hilfe dieses Assays war es möglich, bei gesunden Personen eine mittlere PCT-Serumkonzentration von 13.5 ng/l (13.5 pg/ml) zu bestimmen, wobei Werte zwischen <7 bis 63 ng/l gefunden wurden und das 97.5% Perzentil bei 42.5 ng/l lag.

Angaben zu Versuchen, PCT auch im CSF zu messen, finden sich in der wissenschaftlichen Literatur nur spärlich, und alle beschriebenen Messungen erfolgten unter Prämissen, die sich in keinen logischen Zusammenhang mit der erfindungsgemäßen Bestimmung von PCT in CSF zur Diagnose von Demenz-Erkrankungen und chronischen neuroinflammatorischen Erkrankung nicht-infektiöser Ätiologie gemäß Anspruch 1 bringen lassen:

Ausgehend von der Eignung von PCT als Infektionsmarker wurde versucht, festzustellen, ob PCT-Konzentrationen im CSF von Patienten mit Meningitis (8; 12; 25) oder Lyme Borreliose (14) messbar sind und ggf. eine Unterscheidung zwischen bakterieller Meningitis und viraler Meningitis ermöglichen. Die Befunde waren widersprüchlich, wobei entweder überhaupt keine erhöhten Messwerte erhalten wurden (8; 25) oder nur ein schwacher Nachweis möglich war (12).

Ausgehend von einer genetischen Verwandtschaft zwischen PCT und dem Peptid CGRP (calcitonin-gen-related-peptide) und homologen Sequenzbeziehungen zu Adrenomedullin (ADM), das im CSF von Kindern mit unfallbedingten Gehinverletzungen (traumatic brain injury; TBI) erhöht gemessen wurde, wurde ferner geprüft, ob sich bei solchen Kindern auch erhöhte PCT-Konzentrationen im CSF feststellen lassen (10). Dabei konnten erhöhte PCT-Konzentrationen festgestellt werden, die mit einer Akutphasenreaktion auf das Trauma in Verbindung gebracht wurden, auch wenn die Signifikanz der Beobachtungen insgesamt unklar blieb. Irgendein erkennbarer logischer Zusammenhang zu Messungen bei Demenz-Erkrankungen und weiteren neuroinflammatorischen Erkrankungen, die Gegenstand der vorliegenden Anmeldung sind, ist nicht herstellbar.

In allen Fällen, in denen versucht wurde, PCT im CSF zu bestimmen, wurde mit dem für die Sepsisdiagnostik in Serum oder Plasma entwickelten kommerziellen Assay gearbeitet, der eine FAS von nur 300 ng/l aufwies.

Die Anmelder haben Anlass zu der Annahme, dass auch im Falle von z.B. infektiös bedingten Erkrankungen wie bakterieller Meningitis im CSF erheblich verbesserte Messergebnisse mit besserer diagnostischer Signifikanz erhalten werden, wenn man Messungen, wie sie z.B. in (8; 12) beschrieben werden, mit einem hochsensitiven PCT-Assay gemäß (20) durchführt, wie er bei den Messungen, die Grundlage der vorliegenden Erfindung bilden und im Versuchsteil beschrieben werden, eingesetzt wurde. Dabei ist darauf hinzuweisen, dass mit einem solchen hochsensitiven Assay unter den beschriebenen Bedingungen klare Normkonzentrationen für gesunde Personen ermittelt werden konnten. Es ist daher ein weiterer Gegenstand der vorliegenden Anmeldung, ganz generell für diagnostische Zwecke PCT im CSF mit einem hochsensitiven Immunoassay zu messen.

Nachfolgend wird die Erfindung anhand von Messergebnissen und einer Figur noch näher erläutert.

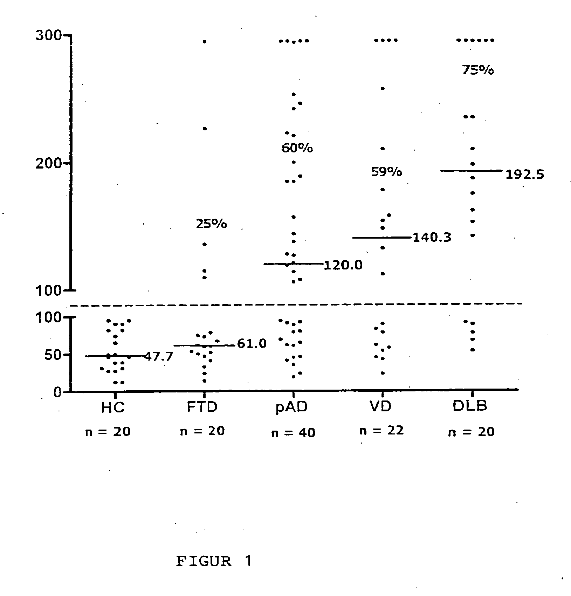

- Figur 1: zeigt die Ergebnisse der Messung der PCT-Immunre- aktivität im CSF von gesunden Normalpersonen (HC) und im CSF von Patienten mit vier verschiedenen diagnostizierten Typen von präsenilen Demenz-Er- krankungen, nämlich frontotemporaler Demenz (FTD), Alzheimer Demenz (pAD), vaskulärer Demenz (VD) und Demenz mit Lewy Körperchen (DLB), und mit den für die gemessenen Patientenkollektive ermittelten medialen Konzentrationen und Sensitivitäten für die einzelnen Demenzformen.

### Experimenteller Teil

### Assavbeschreibung

Die Messung von Procalcitonin im Liquor cerebrospinalis erfolgte wie in (20) beschrieben. Die lyophilisierten Standards wurden jedoch nicht in Nullserum sondern in PBS (mit 1% BSA) gelöst.

### Messung von der PCT-Immunreaktivität im Liquor cerebrospinalis von gesunden Kontrollen und Patienten mit präsenilen Demenzen

Procalcitonin wurde mit dem LUMItest^{®} PCTsensitiv (vgl. 20) in Liquor cerebrospinalis von gesunden Kontrollpersonen nachgewiesen. Es konnte gezeigt werden, dass die Konzentrationen im Bereich zwischen 12 und 133 ng/l liegen (mediale Konzentration 50 ng/l). Da die mediale PCT-Konzentration im Serum von Gesunden nur zu 13,5 ng/ml ermittelt wurde (20), ergibt sich bei gesunden Personen ein PCT-Konzentrationsgradient zwischen Blut und CSF von ca. 1:4.

Die gemessenen PCT-Konzentrationen im Liquor von gesunden Kontrollen und Patienten mit verschiedenen präsenilen Demenzformen sind in Figur 1 gezeigt.

Die jeweilige Sensitivität und Spezifität des hochsensitiven Assays LUMItest^{®} PCTsensitiv für die Diagnose verschiedener präseniler Demenz-Erkrankungen sind in Tabelle 1 angegeben.

**Tabelle 1: Spezifität und Sensitivität der Messungen der PCT-Immunreaktivität im CSF von Patienten mit verschiedenen Demenz-Erkrankungen**

| **Demenz-Erkrankung** | **Spezifität (%)** | **Sensitivität (%)** |
|---|---|---|
| subjektive kognitive Störungen | 100 | 50 |
| frontotemporale Demenz | 100 | 25 |
| Alzheimer-Demenz* | 100 | 60 |
| vaskuläre Demenz | 100 | 59 |
| Demenz mit Lewy-Bodies | 100 | 75 |

| | | |
|---|---|---|
| * Gruppe von Patienten, denen die Diagnose "wahrscheinlich Alzheimer" (wAD; englisch: probable Alzheimer, pAD) gestellt wurde, wobei die diagnostizierende Institution eine mittlere statistische Diagnosezuverlässigkeit der Alzheimer-Diagnose von 90% aufwies. | | |

Die Messergebnisse zeigen gemäß Figur 1 unterschiedliche mediale Konzentrationen für die verschiedenen Patientengruppen, wobei die Gruppe der FTD-Patienten (Patienten mit subjektiven kognitiven Störungen) im Mittel gegenüber Gesunden nur geringfügig erhöhte Messwerte lieferte und sich deutlich von den anderen Patientengruppen unterschied, bei denen die mittleren PCT-Konzentrationen (i) gegenüber Gesunden erheblich erhöht waren, und (ii) sich auch von Gruppe zu Gruppe unterschieden. DLB-Patienten wiesen dabei die höchsten messbaren PCT-Konzentrationen auf und wurden mit einer hohen Sensitivität von 75% (innerhalb der klinisch vorsortierten Gruppen; Spezifität 100%) positiv gefunden.

### Literatur

1. AKIYAMA H. AND THE NEUROINFLAMMATION WORKING GROUP (2000). Inflammation and Alzheimer. Neuroblology of Aging 21: 383-421
2. ASSICOT M., GENDREL D., CARSIN H., RAYMOND J., GUILBAUD J., BOHUON C. (1993). High serum Procalcitonin concentrations in patients with sepsis and infection. Lancet 341: 515-518
3. BECKER K. L., NYLEN E. S., WHITE J. C., MUELLER B., SNIDER R. H. (2004). Procalcitonin and the calcitonin gene family of peptides in inflammation, infection, and sepsis: a journey from calcitonin back to its precursors. Journal of Clinical Endocrinology and Metabolism 89: 1512-1525
4. CAPLAN L., SCHOENE W. C. (1978). Clinical features of subcortical arteriosclerotic encephalopathy (Binswanger's disease). Neurology 28: 1206-1215
5. DANDONA P., NIX D., WILSON M. F., ALJADA A., LOVE J., ASSICOT M., BOHUON C. (1994). Procalcitonin increase after endotoxin injection in normal subjects. Journal of Clinical Endocrinolgy and Metabolism 79: 1605-1608
6. GELDMACHER D. S. (2004). Dementia with lewy bodies: diagnosis and clinical approach. Cleveland clinic Journal of Medicine 71: 789-800
7. GENDREL D., ASSICOT M., RAYMOND J., MOULIN F., FRANCOU-AL C., BADOUAL J., BOHOUN C. (1996). Procalcitonin as a marker for the early diagnosis of neonatal infection. Journal of Pediatrics 128: 570-573
8. GENDREL D., RAYMOND J., ASSICOT M., MOULIN F., INIGUEZ J.-L., LEBON P., BOHUON C. (1997). Measurement of Procalcitonin levels in children with bacterial or viral meningitis. Clinical Infectious Diseases 24: 1240-1242
9. HACHINSKI V. C., LASSEN N. A., MARSHALL J. (1974). Multi-infarct dementia: a cause of mental deterioration in the elderly. Lancet 2: 207-209
10. HAN Y. Y., CARCILLO J. A., RUPPELL R. A., ADELSON P. D., WISNIEWSKI S. R., BELL M. I., JANESKO K. L., MARION D. W., KOCHANEK P. M. (2002). Cerebrospinal fluld Procalcitonin and severe traumatic brain injury in children. Pediatric Critical Care Medicine 3: 39-44
11. HOLMES C., CAIRNS N., LANTOS P., MANN A. (1999). Validity of current clinical criteria for Alzheimer's disease, vascular dementia and dementia with lewy bodies. British Journal of Psychiatry 174: 45-50
12. JEREB M., MULOVIC I., HOJKER S., STRLE F. (2001). Predictive value of serum and cerebrospinal fluid Procalcitonin levels for the diagnosis of bacterial meningitis. Infection 29: 209-212
13. KATSUSE 0., ISEKI E., KOSAKA K. (2003). Immunohistochemical study of the expression of cytokines and nitric oxide synthases in brains of patients with dementia with lewy bodies. Neuropathology 23: 9-15
14. LOTRIC-FURLAN S., MARASPIN-CARMAN V., CIMPERMAN J., ORINE K., STOPAR T., STRLE F. (2002). Procalcitonin levels in patients with Lyme borreliosis. Wiener Klinische Wochenschrift 114: 530-532
15. MACKENZIE I. R. (2000). Activated microglia in dementia with lewy bodies. Neurology 55: 132-134
16. MACKENZIE I. R. (2001). Cortical Inflammation in Dementia With Lewy Bodies, Arch. Neurol. 58: 519-520
17. MCKEITH 1. G., GALASKO D., KOSAKA K., PERRY E. K., DICKSON D. W., HANSEN L. A., SALMON D. P., LOWE J., MIRRA S. S., BYRNE E. J., LENNOX G., QUINN N. P., ED-WARDSON J. A., INCE P. G., BERGERON C., BURNS A., MIL-LER B. L., LOVESTONE S., COLLERTON D., JANSEN E. N., BALLARD C., DE VOS R. A., WILCOCK G. K., JELLINGER K. A., PERRY R. H. (1996). Consensus guidelines for the clinical and pathologic diagnosis of dementia with lewy bodies (DLB): report of the consortium on DLB international workshop. Neurology 47: 1113-1124
18. MCKEITH I. G., O'BRIEN J. T., BALLARD C. (1999). Diagnosing dementia with lewy bodies. Lancet 354: 1227-1228
19. MCKEITH I. G. (2002). Dementia with lewy bodies. British Journal of Psychiatry 180: 144-147
20. MORGENTHALER N. G., STRUCK J., FISCHER-SCHULZ C., BERG-MANN A. (2002). Sensitive immunoluminometric assay for the detection of procalcitonin. Clinical Chemistry 48: 788-789
21. MUELLER B., BECKER K. L., SCHACHINGER H., RICKENBACHER P. R., HUBER P. R., ZIMMERLI W., RITZ R. (2000). Calcitonin precursors are reliable markers of sepsis in a medical intensive care unit. Critical Care Medicine 28: 977-983
22. O'CONNOR E., VENKATESH B., LIPMAN J., MASHONGONYIKA C., HALL J. (2001). Procalcitonin in critical Illness. Critical Care and Resuscitation 3: 236-243
23. RIZZU P., VAN SWIETEN J. C., JOOSSE M., HASEGAWA M., STEVENS M., TIBBEN A., NIERMEIJER M. F., HILLEBRAND M., RAVID R., OOSTRA B. A., GOEDERT M., VAN DUUN C. M., HEUTINK P. (1999). High prevalence of mutations in the microtubule-associated protein tau in a population study of frontotemporal dementia in the Netherlands. American Journal of Human Genetics 64: 414-421
24. SELKOE D. J. (2001). Alzheimer's disease: genes, proteins, and therapy. Physiological Reviews 81: 741-766
25. SHIMETANI N., SHIMETANI K., MORI M. (2001). Levels of three inflammation markers, C-reactive protein, serum amyloid A protein and procalcitonin, in the serum and cerebrospinal fluid of patients with meningitis. Scandinavian Journl of Clinical and Laboratory Investigation 61: 567-574
26. SJOGREN M., FOLKESSON S., BLENNOW K., TARKOWSKI E. (2004). Increased intrathecal inflammatory activity in frontotemporal dementia: pathophysiological implications. Journal of Neurology and Neurosurgical Psychiatry 75: 1107-1111
27. SNIDER R. H. JR., NYLEN E. S., BECKER K. L. (1997). Procalcitonin and its component peptides in systemic inflammation: immunochemical characterization. Journal of Investigative Medicine 45: 552-560
28. TARKOWSKI E. (2002). Cytokines in dementias. Current Drug Targets - Inflammation and Allergy 1: 193-200
29. TARKOWSKI E., LILJEROTH A. M., MINTHON L., TARKOWSKI A., WALLIN A., BLENNOW K. (2003). Cerebral pattern of pro- and anti-inflammatory cytokines in dementias. Brain Research Bulletin 61: 255-260
30. C.E.TEUNISSEN, J. DE VENTE, H.W.M. STEINBUSCH, C. DE BRUIJN (2002), Biochemical markers related to Alzheimer's dementia in serum and cerebrosoinal fluid. Neurobiology of Aging 23, 485-508
31. VARMA A. R., SNOWDEN J. S., LLOYD J. J., TALBOT P. R., MANN D. M., NEARY D. (1999). Evaluation of the NINCDS-ADRDA criteria in the differentiation of Alzheimer's disease and frontotemporal dementia. Journal of Neurology, Neurosurgery and Psychiatry 66: 184-188
32. WHANG K. T., STEINWALD P. M., WHITE J. C., NYLEN E. S., SNIDER R. H., SIMON G. L., GOLDBERG R. L., BECKER K. L. (1998). Serum calcitonin precursors in sepsis and systemic inflammation. Journal of Clinical Endocrinolgy and Metabolism 83: 3296-3301
33. WHICHER I., BIENVENU J., MONNERET G. (2001). Procalcitonin as an acute phase marker. Annals of Clinical Biochemistry 38: 483-893
34. ZHUKAREVA V., VOGELSBERG-RAGAGLIA V., VAN DEERLIN V. M., BRUCE J., I SHUCK T., GROSSMAN M., I CLARK C. M., AR-NOLD S. E., MASLIAH E., GALASKO D., TROJANOWSKI J. Q., LEE V. M. (2001). Loss of brain tau defines novel sporadic and familial tauopathies with frontotemporal dementia. Annals of Neurology 49: 165-175
35. SPENCER CA, LOPRESTI JS, PATEL A, GUTTLER RB, EIGEN A, SHEN D, GRAY D, NICOLOFF JT (1990). Applications of a new chemiluminometric thyrotropin assay to subnormal measurement. J Clin Endocrinol Metab. 1990, 70(2):453-60

## Patentansprüche

1. Liquordiagnostisches in vitro Verfahren zur Erkennung, zur Bestimmung des Schweregrads und zur Verlaufsbeurteilung und Prognose von Demenz-Erkrankungen und von chronischen neuroinflammatorischen Erkrankungen nicht-infektiöser Ätiologie und zur Durchführung im Rahmen der Differentialdiagnostik von Demenz-Erkrankungen, wobei die Demenz-Erkrankungen ausgewählt sind aus einer Gruppe, die die Alzheimer Demenz (AD), Demenz mit Lewy-Körperchen (DLB), frontotemporale Demenz (FTD) und verschiedene Formen der vaskulären Demenz (VD) umfaßt, **dadurch gekennzeichnet, dass** man in einer Probe des Liquor cerebrospinalis (CSF) eines Patienten, der an einer Demenz-Erkrankung oder einer chronischen neuroinflammatorischen Erkrankung nicht-infektiöser Ätiologie leidet oder bei dem Verdacht auf eine solche Erkrankung besteht, eine Bestimmung der Procalcitonin-Immunreaktivität (PCT-Immunreaktivität) durchführt und aus einer gemessenen PCT-Immunreaktivität, die über einem für gesunde Kontrollpersonen typischen Schwellenwert liegt, Schlüsse hinsichtlich des Vorliegens, des Verlaufs, des Schweregrads oder des Erfolgs einer Therapie der Demenz-Erkrankung oder chronischen neuroinflammatorischen Erkrankung nicht-infektiöser Ätiologie zieht.

2. Verfahren nach Anspruch 1, **dadurch gekennzeichnet, dass** man die PCT-Immunreaktivität mit Hilfe eines hochsensitiven PCT-Immunoassays mit einer funktionalen Assay-Sensitivität (FAS) von besser als 100 ng PCT pro l (100 ng/l oder 100 pg/ml), vorzugsweise besser als 10 ng/l, bestimmt.

3. Verfahren nach einem der Ansprüche 1 oder 2, **dadurch gekennzeichnet, dass** man als Schwellenwert für die Diagnose "Verdacht auf chronische neuroinflammtorische Erkrankung nicht-infektiöser Ätiologie" einen für gesunde Kontrollpersonen ermittelten Durchschnittswert heranzieht, der bei etwa 50 pg/ml liegt.

4. Verfahren nach einem der Ansprüche 1 bis 3, **dadurch gekennzeichnet, dass** der PCT-Immunoassay zur Messung der PCT-Immunreaktivität ein Sandwich-Immunoassay unter Verwendung von zwei Antikörpern ist, die an solche Abschnitte des vollständigen PCT-Peptids binden, die bei der proteolytischen Prozessierung von PCT unter Bildung von Calcitonin auf verschiedenen der gebildeten PCT-Teilpeptide liegen oder die auf PCT-Teilpeptiden liegen, die die Calcitonin-Sequenz nicht umfassen.

5. Verfahren nach Anspruch 4, **dadurch gekennzeichnet, dass** einer der Antikörper an einen Abschnitt der Calcitonin-Sequenz bindet und der andere der Antikörper an einen Abschnitt der Katacalcin-Sequenz bindet, und dass wenigstens einer der beiden Antikörper ein affinitätsgereinigter polyklonaler Antikörper ist.

6. Verfahren nach Anspruch 1, **dadurch gekennzeichnet, dass** das Verfahren als differentialdiagnostisches Verfahren durchgeführt wird, bei dem die gemessenen PCT-Immunreaktivitätswerte zu für die einzelnen Demenzformen typischen Wertebereichen in Beziehung gesetzt werden und eine Wahrscheinlichkeit für das Vorliegen einer der möglichen Demenzformen ermittelt wird.

7. Verfahren nach einem der Ansprüche 1 bis 6, **dadurch gekennzeichnet, dass** es im Rahmen einer Multiparameter-Bestimmung durchgeführt wird, bei der gleichzeitig mindestens ein weiterer für das jeweilige Krankheitsbild aussagekräftiger biochemischer oder physiologischer Parameter bestimmt wird und bei der ein Messergebnis in Form eines Satzes von mindestens zwei Messgrößen gewonnen wird, der zur Feindiagnostik der Demenz-Erkrankung oder chronischen neuroinflammtorischen Erkrankung nicht-infektiöser Ätiologie ausgewertet wird.

8. Verfahren nach Anspruch 7, **dadurch gekennzeichnet, dass** im Rahmen der Multiparameter-Bestimmung neben der PCT-Immunreaktivität wenigstens ein weiterer Entzündungsmediator bestimmt wird, der aus den Gruppen der Komplementkomponenten, Cytokine, Chemokine, der Blutkoagulanzien und fibrinolytischen Faktoren, Akutphasenproteine und radikalischen Verbindungen ausgewählt ist.

9. Verfahren nach Anspruch 7 oder 8, **dadurch gekennzeichnet, dass** die Multiparameter-Bestimmung als Simultanbestimmung mittels einer Chiptechnologie-Messvorrichtung oder einer immunchromatographischen Messvorrichtung erfolgt.

10. Verfahren nach einem der Ansprüche 7 bis 9, **dadurch gekennzeichnet, dass** die Auswertung des komplexen Messergebnisses der Multiparameter-Bestimmung mit Hilfe eines Computerprogramms erfolgt.

## Claims

1. CSF diagnostic *in vitro* method for detection, determination of severity and monitoring and prognosis of dementias and chronic neuroinflammatory diseases of non-infectious aetiology and for carrying it out as part of differential diagnosis of dementias, said dementias being selected from a group comprising Alzheimer's dementia (AD), dementia with Lewy bodies (DLB), frontotemporal dementia (FTD) and various forms of vascular dementia (VAD), **characterized in that** a determination of the procalcitonin immunoreactivity (PCT immunoreactivity) is carried out in a sample of the cerebrospinal fluid (CSF) of a patient who is suffering from a dementia or neuroinflammatory disease of non-infectious aetiology or is suspected of suffering from such a disease, and conclusions about the presence, the course, the severity or the success of a treatment of the dementia or neuroinflammatory disease of non-infectious aetiology are drawn from a measured PCT immunoreactivity which is above a threshold value typical for healthy control persons.

2. Method according to Claim 1, **characterized in that** the PCT immunoreactivity is determined with the aid of a highly sensitive PCT immunoassay having a functional assay sensitivity (FAS) of better than 100 ng of PCT per 1 (100 ng/l or 100 pg/ml), preferably better than 10 ng/l.

3. Method according to either of Claims 1 and 2, **characterized in that** an average value which is determined for healthy control persons and is about 50 pg/ml is used as a threshold value for the diagnosis "suspicion of neuroinflammatory disease of non-infectious aetiology".

4. Method according to any of Claims 1 to 3, **characterized in that** the PCT immunoassay for measurement of the PCT immunoreactivity is a sandwich immunoassay using two antibodies which bind to those segments of the complete PCT peptide which are located on different members of the PCT partial peptides formed in the proteolytic processing of PCT with formation of calcitonin or which are located on PCT partial peptides which do not comprise the calcitonin sequence.

5. Method according to Claim 4, **characterized in that** one of the antibodies binds to a segment of the calcitonin sequence and the other of the antibodies binds to a segment of the katacalcin sequence, and **in that** at least one of the two antibodies is an affinity-purified polyclonal antibody.

6. Method according to Claim 1, **characterized in that** the method is carried out as a differential diagnostic method in which the measured PCT immunoreactivity values are related to value ranges typical for the individual forms of dementia and a probability of the presence of one of the possible forms of dementia is determined.

7. Method according to any of Claims 1 to 6, **characterized in that** it is carried out as part of a multi-parameter determination in which at least one further biochemical or physiological parameter informative for the respective clinical picture is simultaneously determined and in which the measured result is obtained in the form of a set of at least two measured variables which is evaluated for the fine diagnosis of dementia or neuroinflammatory disease of non-infectious aetiology.

8. Method according to Claim 7, **characterized in that**, as part of the multi-parameter determination, in addition to the PCT immunoreactivity, at least one further inflammation mediator is determined which is selected from the groups consisting of the complement components, cytokines, chemokines, the blood coagulants and fibrinolytic factors, acute-phase proteins and free radical compounds.

9. Method according to Claim 7 or 8, **characterized in that** the multi-parameter determination is carried out as a simultaneous determination by means of a chip technology measuring apparatus or an immunochromatographic measuring device.

10. Method according to any of Claims 7 to 9, **characterized in that** the evaluation of the complex measured result of the multi-parameter determination is effected with the aid of a computer program.

## Revendications

1. Procédé in vitro de diagnostic du liquide céphalorachidien pour la mise en évidence, la détermination du taux de sévérité et l'évaluation de l'évolution et le pronostic de maladies démentielles et de maladies neuro-inflammatoires chroniques d'étiologie non infectieuse, et à réaliser dans le cadre d'un diagnostic différentiel des maladies démentielles, où les maladies démentielles sont sélectionnées parmi le groupe qui comprend la maladie d'Alzheimer (MA), la démence à corps de Lewy (DCL), la démence fronto-temporale (DFT) et différentes formes de démence vasculaire (DV), **caractérisé en ce que** l'on réalise sur un échantillon de liquide céphalorachidien (LCR) d'un patient, qui soufre d'une maladie démentielle ou d'une maladie neuro-inflammatoire chronique d'étiologie non infectieuse ou chez qui il existe un soupçon d'une telle maladie, la détermination de l'immunoréactivité de la procalcitonine (immunoréactivité PCT) et à partir de l'immunoréactivité PCT mesurée, qui se situe au-dessus d'une valeur seuil typique d'une personne saine de contrôle, on tire des conclusions sur la présence, l'évolution, le taux de sévérité ou les suites d'une thérapie de la maladie démentielle ou de la maladie neuro-inflammatoire chronique d'étiologie non infectieuse.

2. Procédé selon la revendication 1, **caractérisé en ce que** l'on détermine l'immunoréactivité PCT à l'aide d'un immunodosage CT très sensible ayant une sensibilité fonctionnelle du dosage (SFD)supérieure à 100 ng PCT par litre (100 ng/litre ou 100 pg/litre), de préférence supérieure à 10 ng/litre.

3. Procédé selon la revendication 1 ou 2, **caractérisé en ce que** l'on utilise comme valeur seuil pour le diagnostic de « suspicion de maladie neuro-inflammatoire chronique d'étiologie non infectieuse », une valeur moyenne déterminée sur des personnes saines de contrôle, qui se situe àn environ 50 pg/ml.

4. Procédé selon l'une des revendications 1 à 3, **caractérisé en ce que** l'immunodosage PCT pour la mesure de l'immunoréactivité PCT est un immunodosage en sandwiche utilisant deux anticorps, qui se lient à des segments du peptide PCT complet, qui se trouvent sur des parties différentes du peptide PCT formées lors du traitement protéolytique de la PCT avec formation de la calcitonine, ou qui se situent sur des parties peptidiques PCT, qui ne comprennent pas la séquence de la calcitonine.

5. Procédé selon la revendication 4, **caractérisé en ce que** l'un des anticorps se lie à un segment de la séquence de la calcitonine et l'autre des anticorps se lie sur un segment de la séquence de la katacalcine, et **en ce qu'**au moins un des deux anticorps est un anticorps polyclonal purifié par affinité.

6. Procédé selon la revendication 1, **caractérisé en ce que** le procédé est réalisé comme un procédé diagnostique différentiel, où les valeurs mesurées de l'immunoréactivité PCT sont comparées aux plages typiques de valeur pour les formes particulières de démence et on détermine une probabilité pour la présence d'une forme possible de démence.

7. Procédé selon l'une des revendications 1 à 6, **caractérisé en ce qu'**il est réalisé dans le cadre d'une détermination de plusieurs paramètres, dans laquelle au moins un autre paramètre biochimique ou physiologique probant pour les signes cliniques particuliers est simultanément déterminé et dans laquelle un résultat de mesure sous forme d'un ensemble d'au moins deux mesures est obtenu, qui est évalué pour la diagnostic fin de la maladie démentielle ou de la maladie neuro-inflammatoire chronique d'étiologie non infectieuse.

8. Procédé selon la revendication 7, **caractérisé en ce que** dans le cadre d'une détermination à plusieurs paramètres, en plus de l'immunoréactivité PCT, au moins un autre médiateur de l'inflammation est déterminé, lequel est sélectionné dans le groupe des composants du complément, des cytokines, des chimiokines, des facteurs de la coagulation et fibrinolytiques, des protéines de phase aiguë et des composés radicalaires.

9. Procédé selon la revendication 7 ou 8, **caractérisé en ce que** la détermination à plusieurs paramètres est réalisée en tant que détermination simultanée à l'aide d'un dispositif de mesure selon la technologie des puces ou d'un dispositif de mesure immunochromatographique.

10. Procédé selon l'une des revendications 7 à 9, **caractérisé en ce que** l'évaluation des résultats de mesure complexes de la détermination à plusieurs paramètres est réalisée à l'aide d'un programme d'ordinateur.
